# EUROPÄISCHE PATENTSCHRIFT

(11) **EP 1 577 378 B1**
(45) Veröffentlichungstag und Bekanntmachung des Hinweises auf die Patenterteilung: **29.07.2009**
(21) Anmeldenummer: 04006058.4
(22) Anmeldetag: 15.03.2004
(51) Int. Cl.: C12M 1/42, C12M 3/00, C12N 13/00

(54) **Behältnis und Vorrichtung zur Erzeugung von elektrischen Feldern in einzelnen Reaktionsräumen**
Vessel and device for generating electric fields in independent reaction spaces
Récipient et installation destinée a creer des champs électriques dans des espaces de réaction independents

(43) Veröffentlichungstag der Anmeldung: 21.09.2005
(60) Teilanmeldung: 06012478.1
(73) Patentinhaber: Lonza Cologne AG, 50829 Köln (DE)
(72) Erfinder: Müller-Hartmann, Herbert, 50937 Köln (DE); Habig, Michael, 33397 Rietberg (DE)

(56) Entgegenhaltungen:
- WO-A-03/057819
- DE-A- 10 033 578
- US-A- 5 183 744

## Beschreibung

Die Erfindung betrifft ein Behältnis mit Reaktionsräumen, die jeweils zumindest ein aus einer ersten und einer zweiten Elektrode bestehendes Elektrodenpaar zum Anlegen einer elektrischen Spannung zur Erzeugung eines elektrischen Feldes innerhalb des Reaktionsraums aufweisen, wobei zumindest ein Teil der auf der gleichen Seite der unterschiedlichen Reaktionsräume einer Reihe angeordneten ersten Elektroden elektrisch leitfähig gekoppelt sind und mindestens eine zweite Elektrode eines Reaktionsraumes elektrisch separat anschließbar ist, sowie eine Vorrichtung zur elektrischen Kontaktierung mindestens eines entsprechenden Behältnisses, mit Kontaktelementen zum Anlegen einer elektrischen Spannung an die Elektroden des Behältnisses, bei der jeweils ein Kontaktelement für jeweils mindestens eine zweite Elektrode eines Reaktionsraums des Behältnisses vorgesehen ist.

Behältnisse mit mehreren Reaktionsräumen der eingangs genannten Art sind bekannt und werden vor allem bei biochemischen und pharmazeutischen Anwendungen verwendet, wenn eine Vielzahl von Reaktionsansätzen gleichzeitig getestet werden muss. Besondere Anwendungen sind hierbei beispielsweise die Elektroporation, Elektrofusion und Elektrostimulation von lebenden Zellen, sowie alle Anwendungen, bei denen der Reaktionsansatz einem elektrischen Feld ausgesetzt werden muss. Dabei ist das Bestreben eine möglichst große Anzahl von Reaktionsräumen, beispielsweise 96 oder 384, zur Verfügung zu stellen insbesondere bei HT-Analysen (HT = high throughput) zu erkennen, da hier eine Vielzahl von Proben in möglichst kurzer Zeit getestet werden soll. Bei derartigen Behältnissen spricht man in der Regel von Mehrlochpl-atten, Mikrotiterplatten oder Multiwells.

Die bekannten Behältnisse bestehen üblicherweise aus mehreren Reaktionsräumen, die jeweils zwei Elektroden aufweisen, welche mit dem Reaktionsansatz, beispielsweise einer Zellsuspension, im Reaktionsraum in Kontakt stehen. Die beiden Elektroden eines Reaktionsraums erzeugen bei Anlegen einer elektrischen Spannung im Inneren des Reaktionsraums ein elektrisches Feld, wobei sie beispielsweise bei Gleichstrom unterschiedliche Polaritäten aufweisen. Die Elektroden gleicher Polarität, d. h. beispielsweise alle Kathoden und/oder alle Anoden, verschiedener Reaktionsräume sind dabei entweder einstückig ausgebildet oder elektrisch miteinander gekoppelt, so dass sie über einen gemeinsamen elektrischen Kontakt mit der Spannungsquelle verbunden werden können. Solche Anordnungen haben zwar den Vorteil, dass sie einen relativ einfachen Aufbau haben, nachteilig ist hier jedoch, dass die elektrischen Parameter für alle Reaktionsräume gleich sind und somit eine individuelle Ansteuerung der einzelnen Reaktionsräume nicht möglich ist.

Aus der US 2002/0028480 A1 sind beispielsweise Vorrichtungen zur elektrischen Stimulation von lebenden Zellen bekannt. In einer Ausführungsform sind streifenförmige Elektroden paarweise auf den Boden einer Multiwell-Platte aufgebracht. Die streifenförmigen Elektroden ragen dabei jeweils in die einzelnen Reaktionsräume der Platten hinein und stehen somit mit den Reaktionsansätzen in elektrischem Kontakt. Die streifenförmigen Elektroden weisen jeweils an einem ihrer freien Enden eine Kontaktfläche auf, an der ein Spannungsgenerator anschließbar ist. Ein Elektrodenpaar ist dabei jeweils einer Reihe von Reaktionsräumen zugeordnet. In einer besonderen Ausführungsform sind die einen Elektroden, beispielsweise alle Anoden, kurzgeschlossen, während die jeweiligen anderen Elektroden, beispielsweise die Kathoden, einer Reihe einzeln angeschlossen sind. In jedem Fall können bei diesen bekannten Anordnungen aber ausschließlich ganze Reihen von Reaktionsräumen angesteuert werden, d. h. die elektrischen Parameter können folglich nur für einzelne Gruppen von Reaktionsräumen und nicht für jeden einzelnen Reaktionsraum individuell eingestellt werden.

Aus der DE 199 17 571 A1 ist ferner ein Elektrodenraster für Elektroporations-Reaktionsansätze bekannt, das aus einer planen Anordnung von elektrischen Leiterbahnen auf der Oberfläche eines elektrischen Isolators besteht. Die Leiterbahnen stehen sich in Verästelungen gegenüber und schließen jeweils einzelne Reaktionszonen für die Aufnahme der Elektroporations-Reaktionsansätze ein. Die sich gegenüber stehenden Leiterbahnen bilden bei Anlegen einer elektrischen Spannung Elektroden, wobei die einzelnen Leiterbahnen jeweils in einem Hauptast zusammenlaufen, der mit dem Spannungsgenerator verbunden wird. Folglich ist auch mit dieser bekannten Anordnung keine unterschiedliche Einstellung der elektrischen Parameter möglich, so dass alle Reaktionszonen die gleichen elektrischen Bedingungen aufweisen.

Die WO 03/057819 A1 beschreibt unter anderem ebenfalls Multiwell-Platten, bei denen Elektroden unterschiedlicher Reaktionsräume zumindest paarweise elektrisch miteinander verbunden sind, so dass auch hier die elektrischen Parameter nur für einzelne Gruppen von Reaktionsräumen und nicht für jeden einzelnen Reaktionsraum individuell eingestellt werden können.

Die US-A-5 183 744 offenbart eine Vorrichtung zur Behandlung von Zellen mit elektrischem Strom, die ein Behältnis umfasst, das zumindest zwei parallel angeordnete Reihen von Reaktionsräumen mit Elektroden aufweist. Die auf der gleichen Seite der unterschiedlichen Reaktionsräume einer Reihe angeordneten ersten Elektroden sind elektrisch leitfähig gekoppelt und die zweiten Elektroden eines Reaktionsraums sind elektrisch separat anschließbar.

Es ist Aufgabe der Erfindung, ein Behältnis der eingangs genannten Art zu schaffen, dass eine flexible, individuelle und schnelle Erzeugung von elektrischen Feldern in einzelnen Reaktionsräumen eines Behältnisses ermöglicht, sowie eine Vorrichtung zur sicheren und zuverlässigen elektrischen Kontaktierung des Behältnisses zur Verfügung zu stellen.

Die Aufgabe wird erfindungsgemäß durch ein Behältnis der eingangs genannten Art gelöst, bei dem zumindest zwei gegenüberliegend angeordnete erste Elektroden unterschiedlicher Reaktionsräume benachbarter Reihen elektrisch leitfähig gekoppelt sind. Hierdurch kann auf einfache Art und Weise jeder Reaktionsraum des Behältnisses separat ausgewählt und angesteuert bzw. geschaltet werden, so dass die Reaktionsansätze unterschiedlichen Bedingungen ausgesetzt werden können. Es können dadurch mit dem erfindungsgemäßen Behältnis viele Proben in kurzer Zeit unter unterschiedlichen Bedingungen getestet werden, ohne das Behältnis oder die Reaktionsansätze wechseln zu müssen, so dass sich das erfindungsgemäße Behältnis insbesondere für automatisierte HT-Verfahren eignet. Dadurch, dass einige Elektroden elektrisch leitfähig miteinander verbunden sind, kann darüber hinaus der apparative Aufwand hinsichtlich der Kontaktierung der Elektroden verringert werden.

Erfindungsgemäß sind mehrere Reaktionsräume in zumindest zwei parallel angeordneten Reihen angeordnet und zumindest ein Teil der auf der gleichen Seite der unterschiedlichen Reaktionsräume einer Reihe angeordneten Elektroden elektrisch leitfähig gekoppelt. Durch diese besondere Anordnung wird die Herstellung des Behältnisses stark vereinfacht und darüber hinaus durch das Einsparen von Material kostengünstiger. Dadurch, dass zumindest zwei gegenüberliegend angeordnete Elektroden unterschiedlicher Reaktionsräume benachbarter Reihen elektrisch leitfähig gekoppelt sind, kann die Herstellung des erfindungsgemäßen Behältnisses ebenfalls bzw. zusätzlich vereinfacht und kostengünstiger durchgeführt werden.

Zumindest eine Gruppe von gleichnamigen Elektroden oder alle gleichnamigen Elektroden, d. h. beispielsweise diejenigen, die beim Fluss von Gleichstrom die gleiche Polarität aufweisen, und die unterschiedlichen Reaktionsräumen zugeordnet sind, können dabei elektrisch leitfähig gekoppelt sein, was einerseits das Verfahren beschleunigt und andererseits die Kontaktierung der Elektroden vereinfacht.

In Ausgestaltung der Erfindung ist vorgesehen, dass den Elektroden Kontaktflächen zugeordnet sind, die zum Anlegen der elektrischen Spannung elektrisch kontaktierbar sind. Dabei können die zumindest zwei gekoppelten Elektroden unterschiedlicher Reaktionsräume über eine gemeinsame Kontaktfläche kontaktierbar sein, was die Herstellung vereinfacht und die Anzahl der erforderlichen elektrischen Kontakte bzw. Leitungen verringert.

In einer Ausführungsform des erfindungsgemäßen Behältnisses ist vorgesehen, dass die jeweils zweite Elektrode eines Reaktionsraumes eine nur ihr zugeordnete Kontaktfläche aufweist. Durch diese Ausgestaltung des Behältnisses wird sichergestellt, dass die einzelnen Elektroden zur separaten Ansteuerung eines Reaktionsraums unmittelbar und sicher kontaktiert werden können. In einer bevorzugten Ausführungsform der Erfindung ist dabei vorgesehen, dass die Kontaktflächen unmittelbar auf den Elektroden aufgebracht sind.

Die Kontaktflächen können in vorteilhafter Ausgestaltung der Erfindung an der Unterseite der Elektroden im Bodenbereich des Behältnisses angeordnet sein, so dass diese von unten kontaktiert werden können und das Behältnis von oben und von der Seite frei zugänglich bleibt, was insbesondere bei automatisierten Verfahren von Vorteil ist. Dabei kann die Kontaktfläche das untere Ende der Elektrode zumindest annähernd vollständig einnehmen, was die homogene Verteilung des elektrischen Feldes in dem Reaktionsraum positiv beeinflusst, falls die Elektroden aus leitfähigen Polymeren bestehen.

Für besondere Anwendungen, insbesondere für HT-Anwendungen, ist bevorzugt, dass eine Vielzahl von Reaktionsräumen, vorzugsweise 6, 8, 12, 16, 24, 32, 48, 64, 96, 128, 192, 384, 1536, 3456 oder 6144, vorgesehen ist.

Zumindest eine Elektrode kann beispielsweise aus einem Metall, vorzugsweise Gold, Silber oder Aluminium, bestehen.

In besonders vorteilhafter Ausgestaltung der Erfindung ist vorgesehen, dass zumindest eine Elektrode aus einem Polymer besteht, das mit einem elektrisch leitfähigen Stoff, insbesondere Kohlefasern, Graphit, Ruß und/oder Kohlenstoff-Nanotubes, vorzugsweise in einer Konzentration von 40 bis 80 Gew.-%, dotiert ist. Dabei kann das Polymer beispielsweise Polycarbonat, Polyetheretherketon, Polypropylen, Polyamid, vorzugsweise Polyamid 6 oder Polyamid 66, Polyphenylensulfid oder ein Gemisch dieser Polymere sein oder eines oder mehrere dieser Polymere als Hauptbestandteil enthalten. Solche Polymerelektroden sind bei hoher Leitfähigkeit im Spritzgussverfahren herstellbar und geben keine zytotoxischen Stoffe ab, welche die Versuchsergebnisse beeinflussen und verschlechtern könnten.

Die Kontaktfläche besteht in vorteilhafter Ausgestaltung der Erfindung aus einem auf die Elektrode aufgebrachten Kontaktmaterial, welches bei 23°C einen geringeren spezifischen Widerstand oder Grenzflächenwiderstand als das Material aufweist, aus dem die Elektrode besteht. Bevorzugt ist das Kontaktmaterial ein Metall, vorzugsweise Kupfer, oder ein intrinsisch leitender Kunststoff und/oder weist einen spezifischen Widerstand bei 23°C unterhalb von 1 x 10⁻⁵ Ohm·cm, vorzugsweise von 1 x 10⁻⁶ bis 2 x 10⁻⁶ Ohm·cm, auf. Insbesondere bei der Verwendung von Polymerelektroden, die einen relativ hohen Eingangswiderstand aufweisen, kann durch das Kontaktmaterial der Gesamtwiderstand der Elektroden deutlich verringert werden.

Bei dem Verfahren zur Herstellung des Behältnisses wird zunächst ein Wandbereich, der die Reaktionsräume bildet, mit mindestens zwei ausgesparten Bereichen pro Reaktionsraum aus einem nicht leitfähigen Polymer gespritzt und anschließend in die ausgesparten Bereiche ein leitfähiges Material eingebracht oder es werden zunächst pro Reaktionsraum mindestens zwei Bereiche aus einem leitfähigen Material hergestellt oder in eine Form eingesetzt und anschließend wird um die mindestens zwei Bereiche herum ein Wandbereich aus einem nicht leitfähigen Polymer, der die Reaktionsräume bildet, gespritzt, wobei mindestens zwei Bereiche mit leitfähigem Material unterschiedlicher Reaktionsräume elektrisch leitfähig miteinander verbunden werden und der jeweils mindestens eine andere Bereich eines Reaktionsraums mit einer separaten Verbindung zur elektrischen Kontaktierung versehen wird. Durch dieses Verfahren können die erfindungsgemäßen Behältnisse problemlos und kostengünstig hergestellt werden.

In besonders vorteilhafter Ausgestaltung des Herstellungsverfahrens ist vorgesehen, dass zur elektrischen Kontaktierung auf das leitfähige Material ein Kontaktmaterial, vorzugsweise ein Metall oder ein intrinsisch leitender Kunststoff, aufgebracht wird oder dass das Kontaktmaterial auf das leitfähige Material unter Einwirkung von Druck und/oder Wärme, vorzugsweise durch Heißprägen, und/oder über eine haftvermittelnde Schicht, die vorzugsweise einen geringen spezifischen Widerstand aufweist, aufgebracht wird.

Bevorzugt wird bzw. werden bei dem Herstellungsverfahren zumindest eine Gruppe von Reaktionsräumen oder alle Reaktionsräume über jeweils mindestens einen Bereich aus leitfähigem Material elektrisch leitfähig miteinander verbunden.

Als leitfähiges Material können beispielsweise Metallelektroden, vorzugsweise aus Gold, Silber oder Aluminium, in die ausgesparten Bereiche eingesetzt oder in eine Form eingelegt und anschließend umspritzt werden.

In alternativer und bevorzugter Ausführung des Herstellungsverfahrens können als leitfähiges Material auch Polymerelektroden, vorzugsweise aus einem Polymer, das mit einem elektrisch leitfähigen Stoff, insbesondere Kohlefasern, Graphit, Ruß und/oder Kohlenstoff-Nanotubes, vorzugsweise in einer Konzentration von 40 bis 80 Gew.-%, dotiert ist, in eine Form oder die ausgesparten Bereiche eingespritzt werden.

Die Aufgabe wird ferner durch eine Vorrichtung zur elektrischen Kontaktierung der eingangs genannten Art gelöst, bei der ein Kontaktelement für das Anlegen der Spannung an die elektrisch gekoppelten ersten Elektroden von zwei parallel angeordneten Reihen ausreichend ist. Die Kontaktelemente können dabei beispielsweise Stiftkontakte, Federkontakte oder Ähnliches sein.

In vorteilhafter Ausgestaltung der Erfindung ist eine Einrichtung, vorzugsweise eine Platte, vorgesehen, auf der die Kontaktelemente angeordnet sind. Dabei kann die Einrichtung vertikal und/oder horizontal bewegbar, insbesondere mittels eines Motors fahrbar, sein.

Es kann ferner ein Tisch zum Aufsetzen des Behältnisses vorgesehen sein, welcher vorzugsweise horizontal und/oder vertikal bewegbar, insbesondere mittels eines Motors fahrbar, ist. Der Tisch kann mindestens eine Öffnung aufweisen oder eine Lochplatte sein, wobei vorzugsweise die Anzahl der Löcher der Anzahl der Kontaktelemente entspricht. Die Öffnungen oder Löcher dienen dabei dem Hindurchführen der Kontaktelemente zur elektrischen Kontaktierung der Elektroden.

Es kann ferner ein Innenraum, in dem die Kontaktelemente angeordnet sind, und/oder zumindest eine Öffnung vorgesehen sein, durch welche die Kontaktelemente hindurchführbar sind. Auf diese Weise sind die elektrischen Kontakte im Innenraum der Vorrichtung angeordnet, so dass die Sicherheit der bedienenden Personen sichergestellt werden kann.

Es ist bei dieser Ausführungsform vorteilhaft, dass eine Gehäuseöffnung vorgesehen ist, durch die das Behältnis und/oder der Tisch in den Innenraum einschiebbar sind/ist, was die Sicherheit der Vorrichtung weiter erhöht.

Die erfindungsgemäße Vorrichtung kann in vorteilhafter Ausgestaltung zumindest eine elektrische Speichereinrichtung aufweisen oder an mindestens eine elektrische Speichereinrichtung anschließbar sein, wobei die Speichereinrichtung vorzugsweise ein Kondensator ist. Dabei können auch mehrere Speichereinrichtungen parallel oder hintereinander geschaltet sein.

Ferner kann mindestens ein Schaltelement zwischen der Speichereinrichtung und den Elektroden des Behältnisses angeordnet und/oder jedem Reaktionsraum und/oder jeder Gruppe gekoppelter Elektroden ein elektrisches Schaltelement zugeordnet sein, wobei das Schaltelement vorzugsweise ein Relais ist. Durch das Ansteuern der Schaltelemente können dabei die einzelnen Elektroden oder Reaktionsräume gezielt und sicher geschaltet werden.

Es ist ferner vorgesehen, dass die Schaltelemente unmittelbar an den Kontaktelementen angebracht sind, vorzugsweise unterhalb der Einrichtung, auf der die Kontaktelemente angeordnet sind. Diese besonders vorteilhafte Anordnung ermöglicht eine sehr kompakte Bauweise der erfindungsgemäßen Vorrichtung.

Die Erfindung wird im folgenden anhand der Abbildungen beispielhaft näher erläutert.
- Figur 1: zeigt schematisch verschiedene Ansichten einer Ausführungsform eines erfindungsgemäßen Behältnisses zur Durchführung des erfindungsgemäßen Verfahrens,
a) perspektivische Ansicht,
b) Draufsicht von oben,
c) Draufsicht von unten;
- Figur 2: zeigt Draufsichten auf besondere Ausführungsformen erfindungsgemäßer Behältnisse, die als Streifen in einen hierfür vorgesehenen Rahmen einsetzbar sind,
a) teilweise bestückter Rahmen;
b) vollständig bestückter Rahmen;
- Figur 3: zeigt eine perspektivische Ansicht einer Ausführungsform des erfindungsgemäßen Behältnisses mit 96 Reaktionsräumen,
a) gesamte Mikrotiterplatte,
b) gesonderte Darstellung eines Reaktionsraumes;
- Figur 4: zeigt schematisch eine Draufsicht auf die Unterseite des erfindungsgemäßen Behältnisses gemäß Figur 3;
- Figur 5: zeigt eine schematische Darstellung der Anordnung von Kontaktelementen einer erfindungsgemäßen Vorrichtung;
- Figur 6: zeigt eine schematische Darstellung einer weiteren Ausführungsform der Anordnung von Kontaktelementen einer erfindungsgemäßen Vorrichtung:
- Figur 7: zeigt eine perspektivische schematische Darstellung einer erfindungsgemäßen Vorrichtung zur Kontaktierung eines erfindungsgemäßen Behältnisses;
- Figur 8: zeigt Schaltbilder einer Anordnung zur Durchführung des erfindungsgemäßen Verfahrens,
a) Ausführungsform mit gemeinsamer Kathode,
b) Ausführungsform für eine Anordnung der Kontaktelemente,
gemäß Figur 5;
- Figur 9: zeigt ein Flussdiagramm, das die einzelnen Schritte einer Ausführungsform des der Erfindung zugrunde liegenden Verfahrens darstellt.

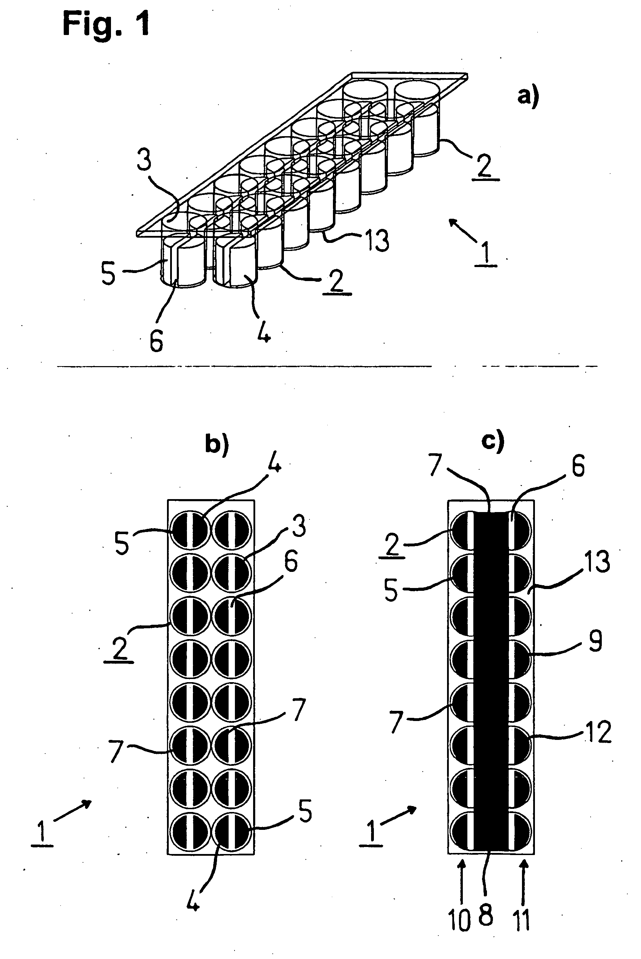

Figur 1 zeigt eine Ausführungsform eines erfindungsgemäßen Behältnisses 1 in verschiedenen Ansichten. Das Behältnis 1 ist in perspektivischer Ansicht a), in einer Draufsicht von oben b) und einer Draufsicht von unten c) dargestellt. Das erfindungsgemäße Behältnis 1 weist 16 Reaktionsräume 2 auf, die jeweils aus einem Wandbereich 3 gebildet sind. Jeder Reaktionsraum 2 umfasst dabei ein Elektrodenpaar, das aus einer ersten Elektrode 4 und einer zweiten Elektrode 5 besteht. Während der Wandbereich 3 aus einem nicht leitfähigen Material, beispielsweise Glas oder Kunststoff, besteht, sind die Elektroden 4, 5 aus einem leitfähigen Material hergestellt. Die Elektroden 4, 5 können dabei beispielsweise aus einem Metall, d. h. Aluminium, Kupfer, Silber oder Gold, bestehen. Bevorzugt sind aber Elektroden, die aus einem Polymer bestehen, das mit einem elektrisch leitfähigen Stoff dotiert ist.

Die Dotierung kann dabei beispielsweise aus Kohlefasern, Graphit, Russ und/oder Kohlenstoff-Nanotubes bestehen und in einer Konzentration von 40 - 80 Gew.-% in dem Polymer vorliegen. Solche leitfähigen Polymere haben den Vorteil, dass sie einfach und kostengünstig im Spritzgussverfahren hergestellt werden können und darüber hinaus im Gegensatz zu Metallelektroden keine zytotoxischen Metallionen freisetzen. Die Konzentration der Dotierung in dem Polymer sollte vorteilhafterweise so gewählt werden, dass die Elektroden einerseits eine ausreichende Leitfähigkeit aufweisen, andererseits aber die Spritzfähigkeit erhalten bleibt. Diese Eigenschaften sind innerhalb des Konzentrationsbereichs von 40 - 80 Gew.-% gewährleistet.

In jedem Reaktionsraum 2 schließen die erste Elektrode 4 und die zweite Elektrode 5 einen Spalt 6 ein, welcher der Aufnahme einer Zellsuspension dient, in der zusätzlich zu den lebenden Zellen biologisch aktive Moleküle, beispielsweise Nukleinsäuren, gelöst sind. Beim Anlegen einer elektrischen Spannung an das Elektrodenpaar fließt durch die Zellsuspension in dem Spalt 6 ein elektrischer Strom, der ein Einschleusen der biologisch aktiven Moleküle in die lebenden Zellen bewirkt. Dieses Verfahren ist unter der Bezeichnung Elektroporation allgemein bekannt. Die beiden Elektroden 4, 5 weisen beim Anlegen einer elektrischen Spannung entgegengesetzte Polaritäten auf, d. h. eine Elektrode 4, 5 weist ein negatives Potential auf und dient somit als Kathode, während die andere Elektrode 4, 5 ein positives Potential aufweist und als Anode dient. Je nach Richtung des Stromflusses können die erste Elektrode 4 und die zweite Elektrode 5 also jeweils entweder als Kathode oder als Anode dienen, wobei die jeweilige Polarität für die vorliegende Erfindung ohne Bedeutung ist. Bei Gleichstrom liegen beispielsweise die entgegengesetzten Polaritäten fest, während diese bei Wechselstrom hin und her schwingen, so dass ein Wechselfeld entsteht.

Zum Herstellen des elektrischen Kontakts sind jeder Elektrode 4, 5 Kontaktflächen 7 zugeordnet. An die Kontaktflächen 7 kann beispielsweise eine Speichereinrichtung, vorzugsweise ein Kondensator, angeschlossen werden, durch dessen gezielte Entladung das elektrische Feld innerhalb des Spalts 6 erzeugt wird. Bei dem in Figur 1 beschriebenen Behältnis 1 sind die Kontaktflächen 7 auf der Unterseite 12 der Elektroden 4, 5 angeordnet, was aus der Ansicht c) deutlich wird. Im vorliegenden Ausführungsbeispiel bestehen die Elektroden 4, 5 aus einem mit leitfähigem Material dotierten Polymer. Die Kontaktflächen 7 bestehen aus einem Kontaktmaterial, das sehr dicht, vorzugsweise unter Einwirkung von Druck und Wärme, d. h. beispielsweise durch Heißprägen, auf das leitfähige Polymer aufgebracht ist. Das Kontaktmaterial weist einen geringeren spezifischen Widerstand (23 °C) bzw. Eintritts- oder Grenzflächenwiderstand als das leitfähige Material auf, aus dem die Elektroden 4, 5 bestehen. Im vorliegenden Ausführungsbeispiel bestehen die Kontaktflächen 7 aus einer Kupferfolie, die mittels Heißprägen auf die Elektroden 4, 5 aufgebracht wurde. Wie aus Ansicht c) ferner deutlich wird, weisen die ersten Elektroden 4, d. h. beispielsweise der Teil der Elektroden des Behältnisses 1, der beim Anlegen einer elektrischen Spannung eine gleichnamige Polarität aufweist, z. B. bei Gleichstrom alle Kathoden, eine gemeinsame Kontaktfläche 8 auf. Die Kontaktfläche 8 besteht aus einem durchgehenden Streifen, der sich über alle ersten Elektroden 4 erstreckt und diese vollständig bedeckt. Auf diese Weise sind alle ersten Elektroden 4 des Behältnisses 1 elektrisch leitfähig gekoppelt. Dies hat den Vorteil, dass das erfindungsgemäße Behältnis 1 einfach und kostengünstig herstellbar ist und die elektrische Kontaktierung der gleichpoligen Elektroden über eine einzige elektrische Verbindung erfolgen kann, was den apparativen Aufwand insgesamt deutlich reduziert. Die jeweils zweite Elektrode 5 eines Reaktionsraums 2 weist eine nur ihr zugeordnete Kontaktfläche 9 auf. Über die einzelnen Kontaktflächen 9 sind also die zweiten Elektroden 5 jedes Reaktionsraumes 2 separat elektrisch anschließbar. Auf diese Weise kann die Erzeugung der elektrischen Felder in jedem Reaktionsraum 2 separat geschaltet werden. Die separate Schaltung jedes Reaktionsraums 2 hat den Vorteil, dass die elektrischen Parameter für jeden Reaktionsraum 2 individuell eingestellt werden können, so dass die einzelnen in den Reaktionsräumen 2 befindlichen Zellsuspensionen bzw. Reaktionsansätze unterschiedlichen Bedingungen ausgesetzt werden können.

Im vorliegenden Ausführungsbeispiel sind die 16 Reaktionsräume 2 in zwei Reihen 10, 11 zu je acht Reaktionsräumen 2 angeordnet. Durch diese reihenförmige Anordnung können die jeweils auf der gleichen Seite einer Reihe 10, 11 angeordneten ersten Elektroden 4 der Reaktionsräume 2 und die jeweils gegenüberliegend angeordneten ersten Elektroden 4 der nebeneinanderliegenden Reihen 10, 11 elektrisch leitfähig gekoppelt werden. Durch diese Anordnung der Reaktionsräume 2 wird die Herstellung des erfindungsgemäßen Behältnisses 1 weiter vereinfacht, Material eingespart und darüber hinaus die elektrische Kontaktierung erleichtert. Dadurch, dass im vorliegenden Ausführungsbeispiel die Kontaktflächen 7, 8, 9 an der Unterseite 12 der Elektroden 4, 5 im Bodenbereich 13 des Behältnisses 1 angeordnet sind, kann die elektrische Kontaktierung von unten erfolgen, was den apparativen Aufwand weiter vereinfacht, insbesondere wenn Behältnisse mit sehr vielen Reaktionsräumen, beispielsweise 96 und mehr, Anwendung finden sollen. Darüber hinaus wird dadurch, dass beide Elektroden 4, 5 von unten kontaktierbar sind, die Verwendung einer von oben in die Zellsuspension eintauchenden Elektrode vermieden, was sich positiv auf die Homogenität des elektrischen Feldes innerhalb des Spaltes 6 auswirkt.

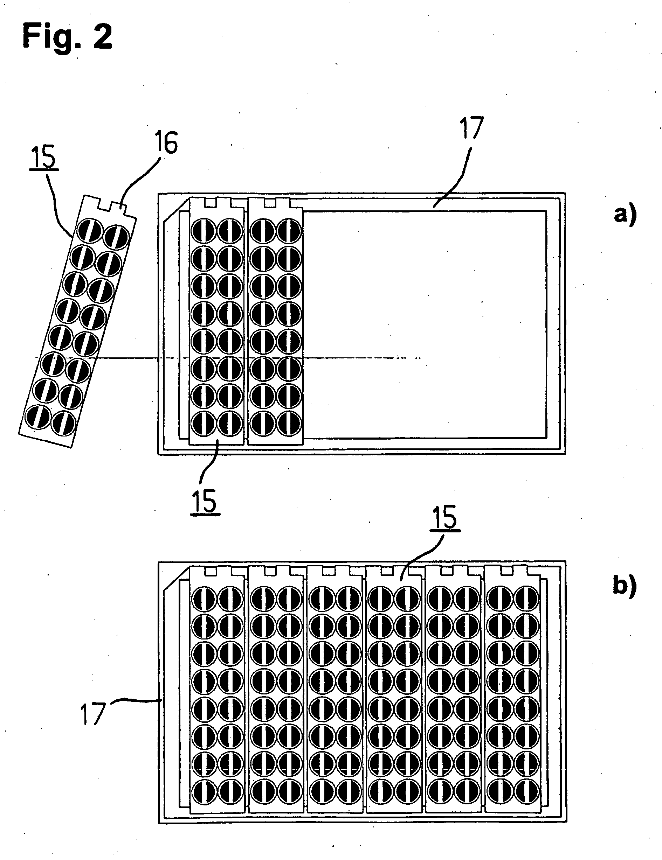

Figur 2 zeigt in Draufsicht eine besondere Ausführungsform erfindungsgemäßer Behältnisse 15, die im Prinzip den Behältnissen 1 gemäß Figur 1 entsprechen. Die Behältnisse 15 sind ebenfalls streifenförmig ausgebildet und weisen an zumindest einem kopfseitigen freien Ende eine besondere Befestigungsvorrichtung 16 auf, die der Befestigung in einem Rahmen 17 dient. Wie in Ansicht a) dargestellt, können die einzelnen Behältnisse 15, die jeweils aus 16 Reaktionsräumen bestehen, einzeln in den Rahmen 17 eingesetzt und wieder entnommen werden. Auf diese Weise kann der Rahmen 17 auch dann wiederverwendet werden, wenn die einzelnen Behältnisse 15 verworfen werden müssen. Hierdurch kann Material eingespart und darüber hinaus die Umweltverträglichkeit des Produktes verbessert werden. Das dargestellte System kann ferner in Abhängigkeit von der Größe des Rahmens 17 flexibel mit einer unterschiedlichen Anzahl von Reaktionsräumen ausgestattet werden. Ansicht b) zeigt einen voll bestückten Rahmen 17, der mit sechs Behältnissen 15 bestückt ist. Auf diese Weise können insgesamt 96 Reaktionsräume genutzt werden, was einer Standard-Mikrotiterplatte entspricht.

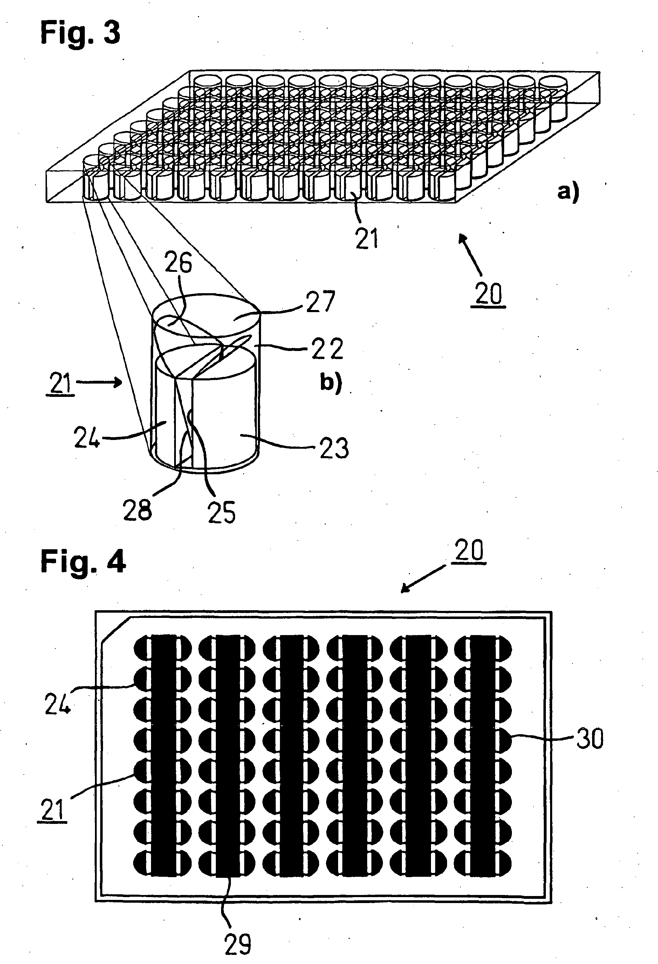

Figur 3 zeigt eine weitere Ausführungsform eines erfindungsgemäßen Behältnisses 20, welches im Prinzip dem Behältnis 1 gemäß Figur 1 entspricht. Im Unterschied zu dem zuvor beschriebenen Behältnis 1 weist das Behältnis 20 jedoch insgesamt 96 Reaktionsräume 21 auf, die in zwölf Reihen zu je acht Reaktionsräumen 21 angeordnet sind. Ein einzelner Reaktionsraum 21 ist in perspektivischer Ansicht b) vergrößert schematisch dargestellt. Der Reaktionsraum 21 besteht aus einem Wandbereich 22 aus einem nicht leitfähigen Polymer, der zylinderförmig ausgebildet ist. Der Reaktionsraum 21 weist ferner ein Elektrodenpaar bestehend aus einer ersten Elektrode 23 und einer zweiten Elektrode 24 auf. Die beiden Elektroden 23, 24 schließen einen Spalt 25 ein, welcher der Aufnahme der Zellsuspension bzw. des Reaktionsansatzes dient. Der Wandbereich 22 des Reaktionsraums 21 ist zum Befüllen des Spalts 25 nach oben hin offen, wobei sich die innere Oberfläche 26 von der Öffnung 27 in Richtung des Spalts 25 nach innen verjüngt. Durch diese trichterförmige Ausgestaltung der Öffnung 27 wird das Befüllen des Reaktionsraums 21 erleichtert, da diese eine Führung für Pipettenspitzen bildet. Der Spalt 25 verengt sich darüber hinaus durch seitliches Zulaufen der Seitenwand 28 nach unten, so dass dieser im Bodenbereich praktisch spitzenförmig endet. Dadurch wird gewährleistet, dass auch geringere Volumina, beispielsweise kleiner als 100 µl, zuverlässig in dem Spalt 25 zwischen die Elektroden 23, 24 aufgenommen werden. Darüber hinaus wird durch diese Ausgestaltung des Spalts 25 ermöglicht, den Reaktionsraum 21 an die Aufnahme unterschiedlicher Volumina anzupassen, ohne die äußeren Dimensionen des Reaktionsraums 21 und damit des gesamten Behältnisses 20 ändern zu müssen, was vor allem dann von Vorteil ist, wenn das Behältnis 20 für automatisierte Prozesse vorgesehen ist. Zur Anpassung des Reaktionsraums 21 an unterschiedliche Volumina muss bei der Herstellung lediglich der Neigungswinkel der Seitenwand 28 verändert werden.

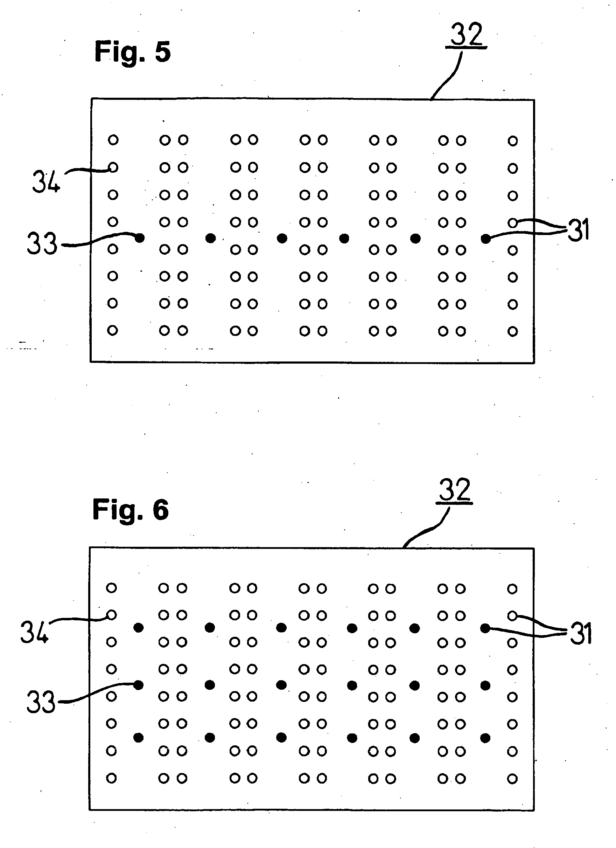

Figur 4 zeigt die Unterseite des erfindungsgemäßen Behältnisses 20 gemäß Figur 3. Analog zu dem erfindungsgemäßen Behältnis 1 gemäß Figur 1 c) weisen auch hier die hier nicht sichtbaren ersten Elektroden, d. h. beispielsweise Elektroden, die beim Anlegen einer elektrischen Gleichspannung die gleiche Polarität aufweisen, zweier benachbarter Reihen eine gemeinsame Kontaktfläche 29 auf. Folglich sind bei diesem Ausführungsbeispiel immer 16 Reaktionsräume über ihre ersten Elektroden elektrisch gekoppelt. Insgesamt weist das Behältnis 20 also sechs gemeinsame Kontaktflächen 29 auf. Die jeweiligen zweiten Elektroden 24 jedes Reaktionsraums 21, die beispielsweise beim Anlegen einer Gleichspannung die entgegengesetzte Polarität aufweisen, sind dagegen ungekoppelt und weisen jeweils eine eigene Kontaktfläche 30 auf. Durch diese besondere Anordnung wird die Herstellung des erfindungsgemäßen Behältnisses 20 vereinfacht, während dennoch ein separates Schalten jedes Reaktionsraums möglich ist. Die elektrische Kopplung mehrerer erster Elektroden eines Behältnisses verringert insgesamt den apparativen Aufwand und ermöglicht darüber hinaus eine effektive und sichere Kontaktierung der Elektroden von der Unterseite, was im folgenden anhand der Figuren 5 und 6 verdeutlicht wird.

In den oben beschriebenen Ausführungsbeispielen sind die Elektroden 4, 5, 23, 24 jeweils halbkreisförmig ausgebildet. Die Elektroden können aber beispielsweise zumindest teilweise auch plattenförmig, d. h. flach, ausgebildet sein. In einer bevorzugten Ausführungsform sind z. B. die Elektroden im Bodenbereich des Behältnisses jeweils halbkreisförmig und weiter oben im Verlauf des Spaltes plattenförmig ausgebildet. Es sind aber auch andere Formen für die Elektroden denkbar.

Figur 5 zeigt eine schematische Darstellung der Anordnung von Kontaktelementen 31 innerhalb einer erfindungsgemäßen Vorrichtung zur Kontaktierung der erfindungsgemäßen Behältnisse. Die Kontaktelemente 31 sind auf oder innerhalb einer besonders hierfür ausgestalteten Einrichtung 32 angeordnet. Bei der Einrichtung 32 kann es sich beispielsweise um eine Platte oder ein Gitter handeln. Die Kontaktelemente 31 können beispielsweise Stiftkontakte, Federkontakte oder Ähnliches sein. Im vorliegenden Ausführungsbeispiel ist die Anordnung der Kontaktelemente 31 auf der Einrichtung 32 derart, dass sie insbesondere zur elektrischen Kontaktierung des erfindungsgemäßen Behältnisses 20 gemäß der Figuren 3 und 4 geeignet ist. Die einzelnen, hier als schwarze Punkte dargestellten Kontaktelemente 33 sind dabei zur Kontaktierung der elektrisch gekoppelten ersten Elektroden bzw. der gemeinsamen Kontaktflächen vorgesehen. Die in Reihen angeordneten, hier weiß dargestellten Kontaktelemente 34 sind dagegen zur Kontaktierung der separat kontaktierbaren zweiten Elektroden vorgesehen. Im vorliegenden Ausführungsbeispiel ist also für jede ungekoppelte zweite Elektrode ein Kontaktelement 34 vorgesehen, während jeweils nur ein Kontaktelement 33 für die elektrisch gekoppelten ersten Elektroden vorgesehen sind. Pro Doppelreihe von Reaktionsräumen wird also nur ein Kontaktelement 33 benötigt. Alle Kontaktelemente 33 können darüber hinaus elektrisch gekoppelt sein. Hierdurch kann die Anzahl der elektrischen Verbindungen deutlich reduziert werden, was die Herstellungskosten und den apparativen Aufwand verringert. Durch die einzelnen, jedem Reaktionsraum zugeordneten Kontaktelemente 34 bleibt dabei die separate Schaltbarkeit jedes Reaktionsraumes erhalten.

Figur 6 zeigt eine alternative Anordnung der Kontaktelemente 31, die derjenigen gemäß Figur 5 mit der Ausnahme entspricht, dass für die gekoppelten Elektroden jeweils insgesamt drei Kontaktelemente 33 vorgesehen sind. Diese drei Kontaktelemente 33 sind in einer Reihe entlang der gemeinsamen Kontaktfläche der gekoppelten zweiten Elektroden angeordnet. Durch das Vorsehen zweier zusätzlicher Kontaktelemente 33 für die gekoppelten Elektroden kann der elektrische Kontakt verbessert und darüber hinaus die Wahrscheinlichkeit einer schlechten oder fehlenden Kontaktierung deutlich verringert werden. Zudem ermöglicht diese Ausführungsform, zwei der drei Kontaktelemente 33 für eine Widerstandsmessung zu verwenden, um die Anwesenheit einzelner Behältnisse 15 innerhalb des Rahmens 17 gemäß Figur 2 zu überprüfen.

Die Kontaktelemente 31 sind auf der Einrichtung 32 gemäß der Figuren 5 und 6 derart angeordnet, dass beispielsweise das erfindungsgemäße Behältnis 20 gemäß der Figuren 3 und 4 einfach mit seiner Unterseite auf die Einrichtung 32 aufgesetzt werden muss, um den elektrischen Kontakt herzustellen. Die Anordnung der Kontaktelemente 31 entspricht dabei exakt der Anordnung der Kontaktflächen 29, 36 der gekoppelten ersten Elektroden 23 und der ungekoppelten zweiten Elektroden 24.

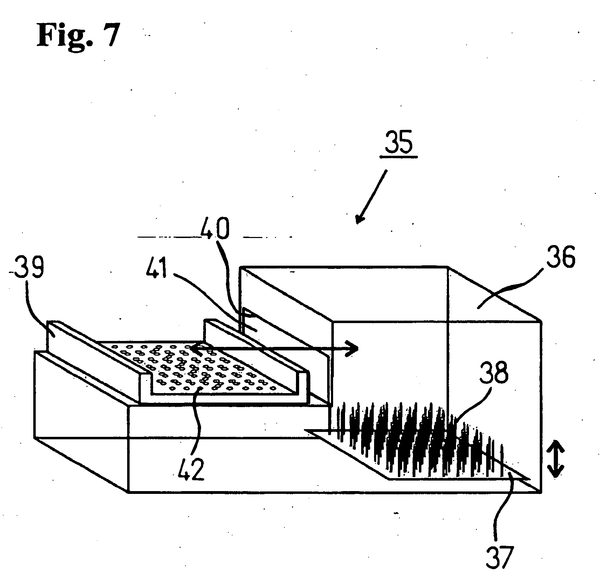

Figur 7 zeigt eine perspektivische schematische Darstellung einer erfindungsgemäßen Vorrichtung 35 zur Kontaktierung der erfindungsgemäßen Behältnisse. Die Vorrichtung 35 besteht aus einem Gehäuse 36, in dem eine Platte 37 angeordnet ist, auf der stiftförmige Kontaktelemente 38 befestigt sind. Die Platte 37 entspricht der Einrichtung 32 gemäß den Figuren 5 und 6. Die Platte 37 mit den Kontaktelementen 38 ist vertikal bewegbar, wobei der zum Verfahren der Platte 37 vorgesehene Mechanismus in dieser Darstellung nicht im einzelnen abgebildet ist. Die Vorrichtung 35 weist ferner einen Tisch 39 auf, welcher horizontal bewegbar ist, wobei auch hier der zum Verfahren des Tisches 39 vorgesehene Mechanismus nicht im einzelnen dargestellt ist. Der Tisch 39 kann durch die Gehäuseöffnung 40 in den Innenraum 41 des Gehäuses 36 gelangen. Das Gehäuse 36 stellt also praktisch eine Art Garage dar, innerhalb der die elektrische Entladung stattfinden kann. Dies ist aus Sicherheitsgründen vorteilhaft, da beispielsweise bei Elektroporationen mit Hochspannungsimpulsen gearbeitet wird, die eine Gefährdung der bedienenden Personen darstellen können. Die bedienende Person braucht hier lediglich das Behältnis mit den Reaktionsansätzen auf den Tisch 39 aufzusetzen, ohne dass ein weiterer Kontakt mit der erfindungsgemäßen Vorrichtung 35 während der weiteren Verfahrensschritte notwendig ist. Wird beispielsweise ein erfindungsgemäßes Behältnis, beispielsweise das Behältnis 20 gemäß der Figuren 3 und 4, auf den Tisch 39 aufgesetzt, so wird anschließend der Tisch 39 mit dem Behältnis durch die Gehäuseöffnung 40 in den Innenraum 41 des Gehäuses 36 der erfindungsgemäßen Vorrichtung 35 gefahren. Dies kann automatisch mittels eines motorgetriebenen Mechanismus erfolgen. Wenn der Tisch 39 mit dem Behältnis sich vollständig im Innenraum 41 befindet, wird die Platte 37 mit den Kontaktelementen 38 nach oben bewegt. Dabei werden die stiftförmigen Kontaktelemente 38 durch die entsprechenden Löcher 42 in dem Tisch 39 hindurchgeführt, so dass sie mit den Kontaktflächen des Behältnisses in Kontakt treten können. Somit ist der elektrische Kontakt zwischen den Kontaktelementen 38 und den Elektroden des Behältnisses hergestellt. Innerhalb des schützenden Gehäuses 36 können dann die Spannungsimpulse zur Erzeugung der elektrischen Felder in den einzelnen Reaktionsräumen des Behältnisses ausgelöst werden.

Die erfindungsgemäße Vorrichtung 35 eignet sich insbesondere auch zur Kontaktierung der erfindungsgemäßen Behältnisse innerhalb vollautomatisierter Verfahren, insbesondere für Verfahren mit sehr hohen Durchsatzzahlen. Dabei wird durch die erfindungsgemäße Vorrichtung 35 eine sichere und zuverlässige Durchführung des Verfahrens gewährleistet. In Abweichung von der hier dargestellten Ausführungsform wäre es alternativ auch möglich, den Tisch mit dem Behältnis außerhalb des Gehäuses zu belassen und nur die Einrichtung mit den Kontaktelementen vertikal und/oder horizontal zu bewegen, um den Kontakt zu den Elektroden herzustellen. Der elektrische Kontakt dürfte in diesem Fall nur hergestellt werden, wenn der Benutzer das Behältnis auf dem Tisch aufgesetzt hat und die Vorrichtung nicht mehr berührt. Da bei vollautomatischen Verfahren das Aufsetzen des Behältnisses nicht von Hand, sondern maschinell erfolgt, wäre diese alternative Ausführungsform durchaus vorteilhaft, da hierdurch ein kompakterer Aufbau der erfindungsgemäßen Vorrichtung möglich wäre.

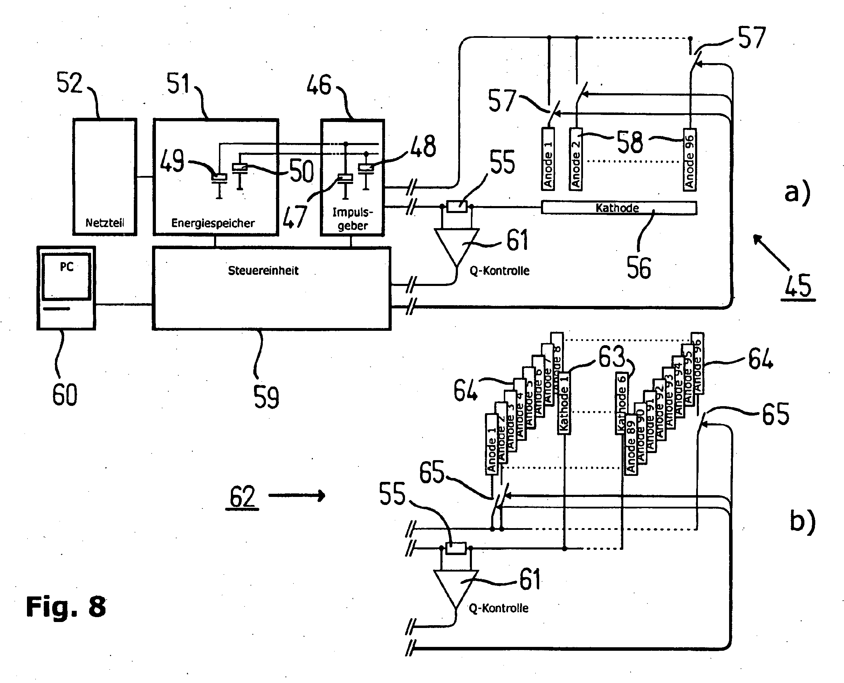

Figur 8 a) zeigt ein schematisches Schaltbild einer Ausführungsform einer erfindungsgemäßen Anordnung 45 zur Durchführung des erfindungsgemäßen Verfahrens. Die Anordnung 45 weist einen Impulsgeber 46 auf, welcher zwei Speichereinrichtungen 47, 48 umfasst. Bei den Speichereinrichtungen 47, 48 handelt es sich jeweils um Kondensatoren, die auf eine vorbestimmte elektrische Ladung aufgeladen werden und durch gezielte Entladung definierte Spannungsimpulse abgeben können. Die Speichereinrichtungen 47, 48 werden durch zusätzliche Speichereinrichtungen 49, 50, bei denen es sich ebenfalls um Kondensatoren handelt, einer Energiespeichervorrichtung 51 auf die vorbestimmte Ladung aufgeladen. Die Speichereinrichtungen 49, 50 werden hierzu durch ein Netzteil 52 gespeist. Das Zwischenschalten zusätzlicher Speichereinrichtungen 49, 50 zwischen das Netzteil 52 und die Speichereinrichtungen 47, 48 hat den Vorteil, dass die Speichereinrichtungen 47, 48 schneller aufgeladen werden können, wodurch schnellere Impulsfolgen möglich sind. Die Speichereinrichtungen 47, 48 sind unmittelbar mit hier nicht dargestellten Leistungshalbleitern verbunden, über welche die gezielte Entladung der Speichereinrichtungen 47, 48 geschaltet wird. Die Leistungshalbleiter können beispielsweise aus einem IGBT oder einem MOSFET bestehen. Es können aber auch andere elektronische Bauelemente verwendet werden, mittels derer die zu schaltenden Spannungen und Ströme mit den erforderlichen Schaltzeiten geschaltet werden können. Die Verwendung von zwei Speichereinrichtungen 47, 48 ermöglicht die Abgabe von zwei kurz aufeinanderfolgenden oder ineinander übergehenden Spannungsimpulsen, was bei der Elektroporation von bestimmten Zelltypen von Vorteil sein kann. Bei diesen besonderen Anwendungen folgt auf einen kurzen Hochspannungsimpuls ein längerer Spannungsimpuls mit geringerer Spannung, wobei beide Spannungsimpulse ineinander übergehen können.

Im vorliegenden Ausführungsbeispiel ist der Impulsgeber 46, d. h. also sowohl die Speichereinrichtung 47 als auch die Speichereinrichtung 48, über einen Messwiderstand 55 mit einer gemeinsamen Elektrode 56 verbunden. Bei der gemeinsamen Elektrode 56 kann es sich beispielsweise um eine Plattenelektrode handeln, die sich im Bodenbereich eines erfindungsgemäßen Behältnisses befindet und sich über alle Reaktionsräume, im vorliegenden Ausführungsbeispiel 96, erstreckt. In diesem Ausführungsbeispiel sind also alle ersten Elektroden eines Behältnisses elektrisch gekoppelt, d. h. weisen beispielsweise beim Anlegen einer elektrischen Gleichspannung die gleiche Polarität auf. Die Speichereinrichtungen 47, 48 sind ferner jeweils über ausschließlich einem Reaktionsraum zugeordnete Schaltelemente 57 mit den jeweiligen zweiten Elektroden 58 des Reaktionsraums verbunden. Bei den Schaltelementen 57 handelt es sich vorzugsweise um Relais. Die zweiten Elektroden 58, die beispielsweise beim Anlegen einer Gleichspannung eine zu der Elektrode 56 entgegengesetzte Polarität aufweisen, sind im vorliegenden Ausführungsbeispiel einzelne stiftförmige Elektroden, die von oben in den Reaktionsraum bzw. die Zellsuspension oder den Reaktionsansatz eintauchen. Da jeder zweiten Elektrode 58 eines der 96 Reaktionsräume ein Relais zugeordnet ist, kann jeder Reaktionsraum separat geschaltet bzw. angesteuert werden, so dass der jeweilige vom Impulsgeber 46 abgegebene Spannungsimpuls ausschließlich zur Erzeugung eines elektrischen Feldes in dem jeweils geschalteten Reaktionsraum führt. Durch das elektrische Koppeln der ersten Elektroden als gemeinsame Elektrode 56 kann dabei der apparative Aufwand, hier insbesondere die Verdrahtung der einzelnen Elektroden, deutlich reduziert werden. Über die 96 Schaltelemente 57 können dabei im vorliegenden Ausführungsbeispiel die 96 Reaktionsräume des Behältnisses separat geschaltet werden, so dass die elektrischen Parameter in jedem Reaktionsraum variiert werden können. Die Schaltelemente 57 werden dabei vorzugsweise sequenziell geschaltet, d. h. die Erzeugung der elektrischen Felder in den einzelnen Reaktionsräumen erfolgt zeitlich nacheinander. Alternativ könnten beispielsweise auch zwei Impulsgeber vorgesehen sein, so dass immer zwei Relais gleichzeitig geschaltet werden könnten, um beispielsweise die Arbeitsgeschwindigkeit insgesamt zu erhöhen. Allerdings müssten dann die Reaktionsräume des Behältnisses praktisch in zwei Bereiche aufgeteilt werden, die jeweils mit einem Impulsgeber zu verbinden wären, was den apparativen Aufwand insgesamt erhöhen würde.

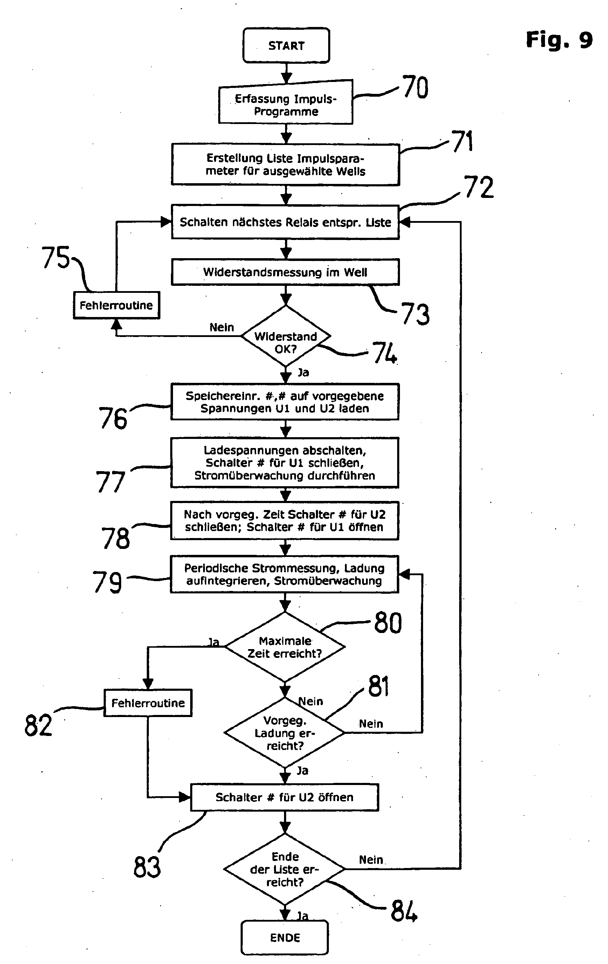

Das Aufladen der Speichereinrichtungen 49, 50 und der Speichereinrichtungen 47, 48 sowie das Schalten der Leistungshalbleiter und der Schaltelemente 57 wird mittels einer Steuereinheit 59 gesteuert. Die Steuereinheit 59 kann beispielsweise über einen herkömmlichen Computer 60 bedient werden. Im vorliegenden Ausführungsbeispiel kann darüber hinaus in vorteilhafter Weise über den Messwiderstand 55 und mittels der Regelungseinheit 61 der Strom während der Abgabe des Spannungsimpulses gemessen werden, so dass durch Aufintegrieren der Ströme in einem bestimmten Zeitintervall eine Kontrolle des Verfahrens aufgrund der insgesamt abgegebenen Ladung ermöglicht wird (Q-Kontrolle). Dabei kann die Steuereinheit 59 beispielsweise veranlassen, dass das jeweilige Relais 57 geöffnet wird, sobald eine vorbestimmte Gesamtladungsmenge erreicht wird. Die Steuerung des erfindungsgemäßen Verfahrens durch die Steuereinheit 59 wird im weiteren näher anhand der Figur 9 beschrieben.

Figur 8 b) zeigt eine weitere Ausführungsform einer Anordnung 62 zur Durchführung des erfindungsgemäßen Verfahrens, welche im wesentlichen der Anordnung 45 gemäß Figur 8 a) entspricht. Die Anordnung 62 unterscheidet sich von der zuvor beschriebenen Anordnung 45 dadurch, dass hier keine gemeinsame erste Elektrode vorgesehen ist, sondern insgesamt sechs Gruppen erster Elektroden 63 elektrisch gekoppelt sind. Die hier gezeigte Ausführungsform eignet sich daher insbesondere zur Verwendung mit dem erfindungsgemäßen Behältnis 20 gemäß der Figuren 3 und 4 bzw. der Einrichtung 32 gemäß Figur 5. Die ersten Elektroden 63 jeweils zweier Reihen von jeweils acht Reaktionsräumen eines Behältnisses, also insgesamt 16 Elektroden, sind hier jeweils elektrisch gekoppelt, d. h. weisen eine gemeinsame Kontaktfläche auf. Der Impulsgeber ist daher in diesem Ausführungsbeispiel hinsichtlich der gekoppelten ersten Elektroden 63 über den Messwiderstand 55 mit insgesamt sechs Kontaktflächen verbunden. Die jeweils zweiten Elektroden 64 jedes Reaktionsraumes sind wiederum jeweils über Schaltelemente 65 mit dem Impulsgeber verbunden. Da bei dieser Ausführungsform jede zweite Elektrode 64 eines Reaktionsraums über ein nur ihr zugeordnetes Schaltelemente 65 separat geschaltet werden kann, kann auch hier die Erzeugung der elektrischen Felder innerhalb jedes der 96 Reaktionsräume separat gesteuert bzw. der jeweils zu schaltende Reaktionsraum frei gewählt werden.

Figur 9 zeigt ein Flussdiagramm, dass die einzelnen Schritte 70 bis 84 einer Ausführungsform des erfindungsgemäßen Verfahrens darstellt. Nach dem Start der Routine werden in Schritt 70 zunächst die Programme für die Spannungsimpulse erfasst. Danach wird in Schritt 71 eine Liste der Impulsparameter für die ausgewählten Reaktionsräume erstellt. In Schritt 72 wird dann das erste bzw. nächste Schaltelement oder entsprechend der jeweiligen Ausführungsform die ersten bzw. nächsten Schaltelemente, das oder die ein separates Schalten des ausgewählten Reaktionsraums bzw. der ausgewählten Reaktionsräume ermöglichen, entsprechend der Liste aus Schritt 71 geschaltet. In Schritt 73 wird dann zunächst der elektrische Widerstand des ausgewählten Reaktionsraums gemessen. In Schritt 74 wird der gemessene Widerstand mit einem Sollwert verglichen. Liegt der gemessene Widerstand nicht innerhalb des Sollbereichs, beispielsweise bei einem leeren Reaktionsraum, so geht die Routine in Schritt 75 zurück zu Schritt 72 und das nächste Schaltelement bzw. die nächsten Schaltelemente der Liste werden geschaltet, d. h. die Routine geht zum nächsten ausgewählten Reaktionsraum über, wobei die Fehlerroutine erneut durchlaufen wird. Liegt der Widerstand innerhalb des Sollbereichs, d. h. ist der Reaktionsraum mit einer korrekten Zellsuspension gefüllt, dann geht die Routine weiter zu Schritt 76. In Schritt 76 wird die Speichereinrichtung bzw. werden die Speichereinrichtungen auf die vorgegebene Spannung U1 bzw. die vorgegebenen Spannungen U1 und U2 geladen. Ist eine Speichereinrichtung vorgesehen, so wird das erfindungsgemäße Verfahren mit einem Spannungsimpuls mit der Spannung U1 durchgeführt. Sind dagegen zwei Speichereinrichtungen vorhanden, so kann das erfindungsgemäße Verfahren auch mit zwei kurz aufeinanderfolgenden und/oder ineinander übergehenden Spannungsimpulsen mit den Spannungen U1 und U2 durchgeführt werden. In Schritt 77 werden die Spannungen zum Aufladen der Speichereinrichtungen abgeschaltet und der Leistungshalbleiter für die erste Speichereinrichtung (U1) wird geschlossen, so dass der ausgewählte Reaktionsraum dem Spannungsimpuls ausgesetzt wird. Der Stromfluss kann dabei über einen Messwiderstand gemessen und überwacht werden. Bei der Verwendung einer Speichereinrichtung bzw. eines Spannungsimpulses geht die Routine direkt zu Schritt 79 oder Schritt 80 über. Alternativ kann auch der Leistungshalbleiter der ersten Speichereinrichtung (U1) nach einer voreingestellten Zeit geöffnet werden, wobei die Routine dann direkt zu Schritt 84 übergeht. Werden dagegen zwei Speichereinrichtungen zur Abgabe von zwei Spannungsimpulsen verwendet, so geht die Routine zunächst zu Schritt 78 über. In Schritt 78 wird nach einer vorher eingestellten Zeit der Leistungshalbleiter der ersten Speichereinrichtung (U1) geöffnet, während der Leistungshalbleiter der zweiten Speichereinrichtung (U2) zur Abgabe des zweiten Spannungsimpulses geschlossen wird. In Schritt 79 wird anhand der periodischen Strommessungen die Ladung aufintegriert und somit die jeweilige Gesamtladung ermittelt. In Schritt 80 wird zunächst festgestellt, ob eine voreingestellte Maximaldauer des Spannungsimpulses bzw. der Spannungsimpulse erreicht ist. Wenn dies der Fall ist, wird der Spannungsimpuls über die Fehlerroutine in Schritt 82 abgebrochen, d. h. die Routine geht zu Schritt 83 über. Ist die maximale Impulsdauer nicht erreicht, so wird in Schritt 81 ermittelt, ob die voreingestellte Gesamtladung erreicht ist. Wenn dies nicht der Fall ist, geht die Routine zurück zu Schritt 79 und durchläuft den Zyklus erneut. Wenn die vorgegebene Gesamtladung erreicht ist, geht die Routine zu Schritt 83 über, in dem der Leistungshalbleiter für die erste Speichereinrichtung (U1) oder ggf. die zweite Speichereinrichtung (U2) geöffnet wird. In Schritt 84 wird dann ermittelt, ob das Ende der Liste erreicht ist. Ist dies nicht der Fall, so geht die Routine zurück zu Schritt 72 und durchläuft den gesamten Zyklus erneut. Wenn das Ende der Liste erreicht ist, wird die Routine beendet. Die Erzeugung der elektrischen Felder in jedem der Reaktionsräume wird also separat geschaltet, wobei die Impulsparameter für jeden Reaktionsraum einzeln voreingestellt und gesteuert werden können. Die Erzeugung der elektrischen Felder in den einzelnen Reaktionsräumen erfolgt dabei sequenziell, wobei die Reihenfolge der ausgewählten Reaktionsräume beliebig voreingestellt werden kann.

### Bezugszeichenliste

- 1: Behältnis
- 2: Reaktionsraum
- 3: Wandbereich
- 4: erste Elektrode
- 5: zweite Elektrode
- 6: Spalt
- 7: Kontaktfläche
- 8: Kontaktfläche
- 9: Kontaktfläche
- 10: Reihe
- 11: Reihe
- 12: Unterseite
- 13: Bodenbereich
- 15: Behältnis
- 16: Befestigungsvorrichtung
- 17: Rahmen
- 20: Behältnis
- 21: Reaktionsraum
- 22: Wandbereich
- 23: erste Elektrode
- 24: zweite Elektrode
- 25: Spalt
- 26: Oberfläche
- 27: Öffnung
- 28: Seitenwand
- 29: Kontaktfläche
- 30: Kontaktfläche
- 31: Kontaktelement
- 32: Einrichtung
- 33: Kontaktelement
- 34: Kontaktelement
- 35: Vorrichtung
- 36: Gehäuse
- 37: Platte
- 38: Kontaktelement
- 39: Tisch
- 40: Gehäuseöffnung
- 41: Innenraum
- 42: Löcher
- 45: Anordnung
- 46: Impulsgeber
- 47: Speichereinrichtung
- 48: Speichereinrichtung
- 49: Speichereinrichtung
- 50: Speichereinrichtung
- 51: Energiespeichervorrichtung
- 52: Netzteil
- 55: Messwiderstand
- 56: Elektrode
- 57: Schaltelement
- 58: Elektrode
- 59: Steuereinheit
- 60: Computer
- 61: Regelungseinheit
- 62: Anordnung
- 63: erste Elektrode
- 64: zweite Elektrode
- 65: Schaltelement
- 70 - 84: Schritt

## Patentansprüche

1. Behältnis (1, 15, 20) mit zumindest zwei parallel angeordneten Reihen von Reaktionsräumen (2, 21), die jeweils zumindest ein aus einer ersten (4, 23) und einer zweiten Elektrode (5, 24) bestehendes Elektrodenpaar zum Anlegen einer elektrischen Spannung zur Erzeugung eines elektrischen Feldes innerhalb des Reaktionsraums (2, 21) aufweisen, wobei zumindest ein Teil der auf der gleichen Seite der unterschiedlichen Reaktionsräume (2, 21) einer Reihe angeordneten ersten Elektroden (4, 23) elektrisch leitfähig gekoppelt sind und mindestens eine zweite Elektrode (5, 24) eines Reaktionsraumes (2, 21) elektrisch separat anschließbar ist, **dadurch gekennzeichnet, dass** zumindest zwei gegenüberliegend angeordnete erste Elektroden (4, 23) unterschiedlicher Reaktionsräume (2, 21) benachbarter Reihen elektrisch leitfähig gekoppelt sind.

2. Behältnis nach Anspruch 1, **dadurch gekennzeichnet, dass** den Elektroden (4, 5, 23, 24) Kontaktflächen (7, 8, 9, 29, 30) zugeordnet sind, die zum Anlegen der elektrischen Spannung elektrisch kontaktierbar sind.

3. Behältnis nach Anspruch 2, **dadurch gekennzeichnet, dass** die zumindest zwei gekoppelten Elektroden (4, 23) unterschiedlicher Reaktionsräume (2, 21) über eine gemeinsame Kontaktfläche (8, 29) kontaktierbar sind.

4. Behältnis nach Anspruch 2 oder 3, **dadurch gekennzeichnet, dass** die jeweils zweite Elektrode (5, 24) eines Reaktionsraumes (2, 21) eine nur ihr zugeordnete Kontaktfläche (9, 30) aufweist.

5. Behältnis nach Anspruch 2, 3 oder 4, **dadurch gekennzeichnet, dass** die Kontaktflächen (7, 8, 9, 29, 30) unmittelbar auf den Elektroden (4, 5, 23, 24) aufgebracht sind.

6. Behältnis nach einem der Ansprüche 2 bis 5, **dadurch gekennzeichnet, dass** die Kontaktflächen (7, 8, 9, 29, 30) an der Unterseite der Elektroden (4, 5, 23, 24) im Bodenbereich (13) des Behältnisses (1, 15, 20) angeordnet sind.

7. Behältnis nach einem der Ansprüche 1 bis 6, **dadurch gekennzeichnet, dass** eine Vielzahl von Reaktionsräumen (2, 21), vorzugsweise 6, 8, 12, 16, 24, 32, 48, 64, 96, 128, 192, 384, 1536, 3456 oder 6144, vorgesehen ist.

8. Behältnis nach einem der Ansprüche 1 bis 7, **dadurch gekennzeichnet, dass** zumindest eine Elektrode (4, 5, 23, 24) aus einem Polymer besteht, das mit einem elektrisch leitfähigen Stoff, insbesondere Kohlefasern, Graphit, Ruß und/oder Kohlenstoff-Nanotubes, vorzugsweise in einer Konzentration von 40 bis 80 Gew.-%, dotiert ist.

9. Behältnis nach einem der Ansprüche 2 bis 8, **dadurch gekennzeichnet, dass** die Kontaktfläche (7, 8, 9, 29, 30) aus einem auf die Elektrode (4, 5, 23, 24) aufgebrachten Kontaktmaterial besteht, welches bei 23°C einen geringeren spezifischen Widerstand oder Grenzflächenwiderstand als das Material aufweist, aus dem die Elektrode (4, 5, 23, 24) besteht.

10. Behältnis nach Anspruch 9, **dadurch gekennzeichnet, dass** das Kontaktmaterial ein Metall, vorzugsweise Kupfer, oder ein intrinsisch leitender Kunststoff ist und/oder dass das Kontaktmaterial einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweist.

11. Vorrichtung (35) zur elektrischen Kontaktierung mindestens eines Behältnisses (1, 15, 20) nach einem der Ansprüche 1 bis 10 mit Kontaktelementen (31, 34, 38) zum Anlegen einer elektrischen Spannung an die Elektroden (4, 5, 23, 24) des Behältnisses (1, 15, 20), bei der jeweils ein Kontaktelement (31, 34, 38) für jeweils mindestens eine zweite Elektrode (5, 24) eines Reaktionsraums (2, 21) des Behältnisses (1, 15, 20) vorgesehen ist, **dadurch gekennzeichnet, dass** ein Kontaktelement (31, 33) für das Anlegen der Spannung an die elektrisch gekoppelten ersten Elektroden (4, 23) von zwei parallel angeordneten Reihen ausreichend ist.

12. Vorrichtung nach Anspruch 11, **dadurch gekennzeichnet, dass** ein Innenraum (41) vorgesehen ist, in dem die Kontaktelemente (38) angeordnet sind.

13. Vorrichtung nach Anspruch 11 oder 12, **dadurch gekennzeichnet, dass** diese zumindest eine elektrische Speichereinrichtung (47, 48, 49, 50) aufweist oder an mindestens eine elektrische Speichereinrichtung (47, 48, 49, 50) anschließbar ist, wobei die Speichereinrichtung (47, 48, 49, 50) vorzugsweise ein Kondensator ist.

14. Vorrichtung nach einem der Ansprüche 11 bis 13, **dadurch gekennzeichnet, dass** mindestens ein Schaltelement (57, 65) zwischen der Speichereinrichtung (47, 48, 49, 50) und den Elektroden (4, 5, 23, 24) des Behältnisses (1, 15, 20) angeordnet ist und/oder dass jedem Reaktionsraum (2, 21) und/oder jeder Gruppe gekoppelter Elektroden (4, 23) ein elektrisches Schaltelement (57, 65) zugeordnet ist, wobei das Schaltelement (57, 65) vorzugsweise ein Relais ist.

15. Vorrichtung nach Anspruch 14, **dadurch gekennzeichnet, dass** die Schaltelemente (57, 65) unmittelbar an den Kontaktelementen (31, 33, 34, 38) angebracht sind, vorzugsweise unterhalb der Einrichtung (32), auf der die Kontaktelemente (31, 33, 34, 38) angeordnet sind.

## Claims

1. A vessel (1, 15, 20) with at least two rows of reaction spaces (2, 21) arranged in parallel, each of which has at least one electrode pair, consisting of a first (4, 23) and a second electrode (5, 24), for purposes of applying an electric voltage to generate an electric field within the reaction space (2, 21), wherein at least a proportion of the first electrodes (4, 23) arranged on the same side of the different reaction spaces (2, 21) of one row are coupled in an electrically conducting manner, and at least one second electrode (5, 24) of a reaction space (2, 21) can be connected in an electrically separate manner, **characterised in that** at least two first electrodes (4, 23) arranged opposite one another of different reaction spaces (2, 21) of adjacent rows are coupled in an electrically conducting manner.

2. The vessel according to Claim 1, **characterised in that** contact surfaces (7, 8, 9, 29, 30) are assigned to the electrodes (4, 5, 23, 24), with which surfaces contact can be made for the purposes of applying the electric voltage.

3. The vessel according to Claim 2, **characterised in that** contact can be made with the at least two coupled electrodes (4, 23) of different reaction spaces (2, 21) via a common contact surface (8, 29).

4. The vessel according to Claim 2 or 3, **characterised in that** each of the second electrodes (5, 24) of a reaction space (2, 21) has a contact surface (9, 30) that is assigned only to that electrode.

5. The vessel according to Claim 2, 3 or 4, **characterised in that** the contact surfaces (7, 8, 9, 29, 30) are applied directly onto the electrodes (4, 5, 23, 24).

6. The vessel according to one of the Claims 2 to 5, **characterised in that** the contact surfaces (7, 8, 9, 29, 30) are arranged on the underside of the electrodes (4, 5, 23, 24) in the floor region (13) of the vessel (1, 15, 20).

7. The vessel according to one of the Claims 1 to 6, **characterised in that** a multiplicity of reaction spaces (2, 21) is provided, preferably 6, 8, 12, 16, 24, 32, 48, 64, 96, 128, 192, 384, 1536, 3456 or 6144.

8. The vessel according to one of the Claims 1 to 7, **characterised in that** at least one electrode (4, 5, 23, 24) consists of a polymer, which is dosed with an electrically conducting material, in particular carbon fibres, graphite, particulate carbon and/or carbon nanotubes, preferably in a concentration of 40 to 80 % by weight.

9. The vessel according to one of the Claims 2 to 8, **characterised in that** the contact surface (7, 8, 9, 29, 30) consists of a contact material applied onto the electrode, which material has a lower specific resistance or boundary resistance at 23°C than the material of which of the electrode (4, 5, 23, 24) consists.

10. The vessel according to Claim 9, **characterised in that** the contact material is a metal, preferably copper, or an intrinsically conducting plastic and/or **in that** the contact material has a specific resistance at 23°C of less than 1 x 10⁻⁵ ohm·cm, preferably from 1 x 10⁻⁶ to 2 x 10⁻⁶ ohm·cm.

11. A device (35) for the purposes of making electrical contact with at least one vessel (1, 15, 20) according to one of the Claims 1 to 10, with contact elements (31, 34, 38) for the purposes of applying an electric voltage to the electrodes (4, 5, 23, 24) of the vessel (1, 15, 20), in which one contact element (31, 34, 38) is provided in each case for at least one second electrode (5, 24) in each case of a reaction space (2, 21) of the vessel (1, 15, 20), **characterised in that** one contact element (31, 33) is sufficient for the application of the voltage to the electrically coupled first electrodes (4, 23) of two rows arranged in parallel.

12. The device according to Claim 11, **characterised in that** an interior space (41) is provided in which the contact elements (38) are arranged.

13. The device according to Claim 11 or 12, **characterised in that** this has at least one electrical storage device (47, 48, 49, 50), or can be connected to at least one electrical storage device (47, 48, 49, 50), wherein the storage device (47, 48, 49, 50) is preferably a condenser.

14. The device according to one of the Claims 11 to 13, **characterised in that** at least one circuit element (57, 65) is arranged between the storage device (47, 48, 49, 50) and the electrodes (4, 5, 23, 24) of the vessel (1, 15, 20) and/or **in that** an electrical circuit element (57, 65) is assigned to each reaction space (2, 21) and/or each group of coupled electrodes (4, 23), wherein the circuit element (57, 65) is preferably a relay.

15. The device according to Claim 14, **characterised in that** the circuit elements (57, 65) are fitted directly onto the contact elements (31, 33, 34, 38), preferably underneath the device (32) on which the contact elements (31, 33, 34, 38) are arranged.

## Revendications

1. Récipients (1, 15, 20) comprenant au moins deux rangées disposées en parallèle d'espaces de réaction (2, 21), qui présentent chacun au moins une paire d'électrodes constituée d'une première électrode (4, 23) et d'une seconde électrode (5, 24), pour l'application d'une tension électrique pour générer un champ électrique à l'intérieur de l'espace de réaction (2, 21), une partie des premières électrodes (4, 23) disposée sur le même côté des différents espaces de réaction (2, 21) d'une rangée étant couplée de façon électroconductrice et au moins une seconde électrode (5, 24) d'un espace de réaction (2, 21) pouvant être raccordée séparément au plan électrique, **caractérisé en ce qu'**au moins deux premières électrodes (4, 23), disposées l'une en face de l'autre, de différents espaces de réaction (2, 21) de rangées voisines sont couplées de façon électroconductrice.

2. Récipient selon la revendication 1, **caractérisé en ce que** des surfaces de contact (7, 8, 9, 29, 30), qui peuvent être mises en contact électriquement pour l'application de la tension électrique, sont attribuées aux électrodes (4, 5, 23, 24).

3. Récipient selon la revendication 2, **caractérisé en ce que** les au moins deux électrodes (4, 23) couplées de différents espaces de réaction (2, 21) peuvent être mises en contact au moyen d'une surface de contact (8, 29) commune.

4. Récipient selon la revendication 2 ou 3, **caractérisé en ce que** la respectivement seconde électrode (5, 24) d'un espace de réaction (2, 21) présente une surface de contact (9, 30) attribuée uniquement à cette électrode.

5. Récipient selon la revendication 2, 3 ou 4, **caractérisé en ce que** les surfaces de contact (7, 8, 9, 29, 30) sont appliquées directement sur les électrodes (4, 5, 23, 24).

6. Récipient selon l'une quelconque des revendications 2 à 5, **caractérisé en ce que** les surfaces de contact (7, 8, 9, 29, 30) sont disposées sur le côté inférieur des électrodes (4, 5, 23, 24) dans la zone de fond (13) du récipient (1, 15, 20).

7. Récipient selon l'une quelconque des revendications 1 à 6, **caractérisé en ce qu'**une pluralité d'espaces de réaction (2, 21), de préférence 6, 8, 12, 16, 24, 32, 48, 64, 96, 128, 192, 384, 1536, 3456 ou 6144, est prévue.

8. Récipient selon l'une quelconque des revendications 1 à 7, **caractérisé en ce qu'au** moins une électrode (4, 5, 23, 24) est à base d'un polymère qui est dopé avec une substance électroconductrice, en particulier des fibres de carbone, du graphite, de la suie et/ou des nanotubes de carbone, de préférence dans une concentration de 40 à 80 % en poids.

9. Récipient selon l'une quelconque des revendications 2 à 8, **caractérisé en ce que** la surface de contact (7, 8, 9, 29, 30) est à base d'un matériau de contact appliqué sur l'électrode (4, 5, 23, 24), lequel présente à 23°C une résistance ou une résistance de surface limite spécifique plus faible que le matériau dans lequel l'électrode (4, 5, 23, 24) est faite.

10. Récipient selon la revendication 9, **caractérisé en ce que** le matériau de contact est un métal, de préférence du cuivre, ou une matière synthétique prise intrinsèquement conductrice et/ou **en ce que** le matériau de contact présente une résistance spécifique à 23° C inférieure à 1 x 10⁻⁵ Ohm·cm, de préférence de 1 x 10⁻⁶ jusqu'à 2 x 10⁻⁶ Ohm·cm.

11. Dispositif (35) pour la mise en contact électrique d'au moins un récipient (1, 15, 20) selon l'une quelconque des revendications 1 à 10 avec des éléments de contact (31, 34, 38) pour l'application d'une tension électrique sur les électrodes (4, 5, 23, 24) du récipient (1, 15, 20) à laquelle à chaque fois un élément de contact (31, 34, 38) est prévu pour respectivement au moins une seconde électrode (5, 24) d'un espace de réaction (2, 21) du récipient (1, 15, 20), **caractérisé en ce qu'**un élément de contact (31, 33) est suffisant pour l'application de la tension sur les premières électrodes (4, 23) couplées électriquement de deux rangées disposées en parallèle.

12. Dispositif selon la revendication 11, **caractérisé en ce qu'**il est prévu un espace intérieur (41) dans lequel les éléments de contact (38) sont disposés.

13. Dispositif selon la revendication 11 ou 12, **caractérisé en ce que** celui-ci présente au moins un dispositif accumulateur (47, 48, 49, 50) électrique ou peut être raccordé à au moins un dispositif accumulateur (47, 48, 49, 50) électrique, le dispositif accumulateur (47, 48, 49, 50) étant de préférence un condensateur.

14. Dispositif selon l'une quelconque des revendications 11 à 13, **caractérisé en ce qu'**au moins un élément de commutation (57, 65) est disposé entre le dispositif accumulateur (47, 48, 49, 50) et les électrodes (4, 5, 23, 24) du récipient (1, 15, 20) et/ou **en ce qu'**un élément de commutation (57, 65) électrique est attribué à chaque espace de réaction (2, 21) et/ou chaque groupe d'électrodes (4, 23) couplées, l'élément de commutation (57, 65) étant de préférence un relais.

15. Dispositif selon la revendication 14, **caractérisé en ce que** les éléments de commutation (57, 65) sont placés directement sur les éléments de contact (31, 33, 34, 38), de préférence au-dessous du dispositif (32) sur lequel les éléments de contact (31, 33, 34, 38) sont disposés.
